# FASCICULE DE BREVET EUROPEEN

(11) **EP 0 707 571 B1**
(45) Date de publication et mention de la délivrance du brevet: **02.05.1997**
(21) Numéro de dépôt: 94920515.7
(22) Date de dépôt: 27.06.1994
(51) Int. Cl.: C07D 253/06, A61K 31/53, C07D 403/12, C07D 405/12

(54) **DERIVES DE LA 3,5-DIOXO-(2H,4H)-1,2,4-TRIAZINE COMME 5HT1A LIGANDS**
3,5-DIOXO-(2H,4H)-1,2,4-TRIAZINE ALS 5HT1A LIGANDE
3,5-DIOXO-(2H,4H)-1,2,4-TRIAZINE DERIVATIVES AS 5HT1A LIGANDS

(30) Priorité: 06.07.1993 FR 9308259
(43) Date de publication de la demande: 24.04.1996
(73) Titulaire: PIERRE FABRE MEDICAMENT, 92100 Boulogne (FR)
(72) Inventeur: PATOISEAU, Jean-François, F-81100 Castres (FR); COURET, Françoise, F-31450 Corransac (FR); FAURE, Christian, F-31200 Toulouse (FR); DUPONT-PASSELAIGUE, Elisabeth, F-81100 Castres (FR); KOEK, Wouter, F-81290 Viviers-les-Montagnes (FR)
(74) Mandataire: Doat, Jean Pierre
(86) Numéro de dépôt international: FR9400772
(87) Numéro de publication internationale: WO9501965

(56) Documents cités:
- EP-A- 0 478 954
- EP-A- 0 512 755
- EP-A- 0 527 081
- EP-A- 0 559 285
- WO-A-92/06082
- JOURNAL OF MEDICINAL CHEMISTRY, vol.35, no.13, 26 Juin 1992, WASHINGTON US pages 2369 - 2374 JERZY L. MOKROSZ ET AL. 'Structure-activity relationship studies of central nervous system agents. 5. Effect of ...'

## Description

La présente invention a pour objet de nouveaux dérivés de la 3,5-dioxo-6 amino-(2H,4H)-1,2,4 triazine, leur préparation et leur application thérapeutique.

Dans le cadre de la recherche de nouveaux médicaments anxiolytiques à profil non benzodiazépinique, la découverte et le développement de la buspirone ont suscité un nombre important de travaux. De fait, ces dernières années, de nombreux composés possèdant une affinité vis-à-vis des récepteurs 5HT_{1A} ont été revendiqués pour leur activité anxiolytique et/ou antihypertensive (J. Peergaard et al. Current opinion in therapeutic Patents, Janvier 1993, 101-128).

Les composés de la présente invention se caractérisent par leur structure originale, leur puissante affinité vis-à-vis du récepteur 5HT_{1A} et leur profil pharmacologique.

Les composés de l'invention correspondent à la formule générale I 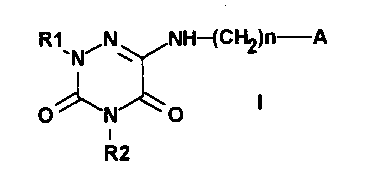 dans laquelle :
- R₁ et R₂, identiques ou différents représentent l'hydrogène ou un radical alcoyle en C₁-C₆
- n peut prendre les valeurs entières de 2 à 6
- A représente un groupement de type
   . aryl piperazino II 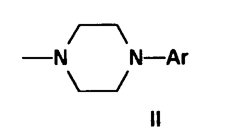 le groupement Ar représentant lui-même une structure aromatique tel que phényle, naphtyle, pyrimidyle, pyridyle, éventuellement substituée par un ou plusieurs groupements tels que alcoyle en C₁-C₃, alcoxy en C₁-C₃, hydroxy, trifluorométhyle ou halogène.
   . benzodioxanyl méthyl amino ou pyridodioxanyl méthyl amino III 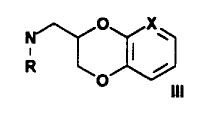 dans lequel R représente l'hydrogène ou un groupement alcoyle en C₁-C₃ et X représente un atome d'azote ou de carbone.

En outre, l'invention couvre les sels des composés de formule générale I avec les acides pharmaceutiquement acceptables dans le cas des composés présentant une basicité suffisante, ainsi que les différents énantiomères pour les composés possédant un carbone asymétrique.

Les composés de l'invention peuvent être obtenus par un procédé chimique caractérisé en ce que l'on condense un composé de formule générale IV. 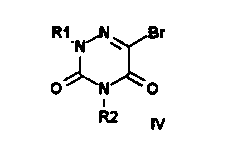 avec un composé de formule génerale V.

H₂N-(CH2)n-A V

les radicaux R₁, R₂, n, A ayant la même signification dans IV et V que dans la formule générale I.

Cette réaction peut être effectuée au reflux du butanol en présence d'une base telle que la triéthylamine.

Les composés de formule générale IV sont eux-mêmes obtenus selon l'invention tel que décrit ci-après :
a) lorsque R₁ = R₂ = H, par bromation de la 3,5-dioxo-(2H,4H)-1,2,4 triazine par le brome en milieu aqueux.
b) lorsque R₁ = R₂ = alcoyle, par alcoylation de la 3,5-dioxo-(2H,4H)-1,2,4-triazine en présence d'hydrure de sodium dans le DMF par un halogénure d'alcoyle puis bromation selon la même méthode que a) précédemment. Il est nécessaire, pour l'alcoylation d'isoler intermédiairement le mélange de composés monoalcoylés formés et de renouveler l'opération d'alcoylation dans les mêmes conditions pour avoir une réaction complète.
c) lorsque R₁ = H et R₂ = alcoyle à partir de la 3,5-dioxo-(2H,4H)1,2,4-triazine par :
   1- acétylation en position 2 par traitement à l'anhydride acétique ou le chlorure d'acétyle
   2 - alcoylation de la position 4 par un halogénure d'alcoyle R₂X en présence de NaH dans le DMF, X représentant Cl, Br ou I
   3 -désacétylation en milieu acide tel que l'acide p.toluène sulfonique dans l'éthanol
   4 - bromation selon la méthode décrite précédemment.
d) lorsque R₁ ≠ R₂ = alcoyle, par alcoylation du composé obtenu selon c/3 par un halogénure d'alcoyle R₁X en présence de NaH dans le DMF, X représentant Br, Cl ou I puis bromation tel que décrit précédemment.
e) lorsque R₁ = alcoyle et R₂ = H, par :
   1 - synthèse de la 3-thiooxo-5-oxo-(2H,4H)-1,2,4 triazine par condensation de l'acide glyoxylique sur le thiosemicarbazide suivi d'un traitement basique
   2 - méthylation par l'iodure de méthyle en présence de NaH dans le DMF
   3 - alcoylation en position 2 par un halogénure d'alcoyle R₁X en présence de NaH dans le DMP, X représentant Cl, Br ou I
   4 - traitement en milieu acide tel que l'acide chlorhydrique
   5 - bromation selon la méthode décrite précédemment.

La préparation des composés I pour lesquels R₁ = R₂ = H peut également être effectuée de façon avantageuse en condensant l'amine V sur le composé bromé et acétylé VI 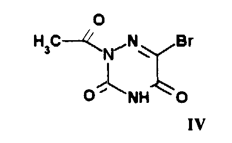 puis en traitant le dérivé obtenu en milieu acide tel que l'acide chlorhydrique ou l'acide p. toluène sulfonique.

Le composé VI est lui même obtenu par acétylation à l'anhydride acétique ou au chlorure d'acétyle de la 6-bromo-3,5-dioxo-(2H,4H)-1,2,4 triazine.

Les composés V sont des amines commerciales ou peuvent être obtenus de façon classique telle que génératon de l'amine primaire à partir du phtalimide intermédiaire.

Les exemples suivants illustrent l'invention sans en limiter la portée. Les analyses élémentaires et les spectres IR et RMN confirment les structures des composés obtenus selon l'invention.

### Exemple 1 :

### 2,4-diméthyl-3,5-dioxo-(2H,4H)-6-(4-(4-(3-trifluorométhylphényl)pipérazino) butylamino)-1,2,4-triazine 1.

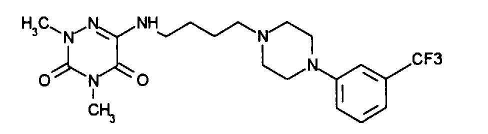

a) 2,4-diméthyl-3,5-dioxo(2H,4H)-1,2,4-triazine 1a.
   A une suspension d'hydrure de sodium à 60 % dans l'huile de parafine (8,8 g ; 0,22 mole) dans le DMF (100 ml) est ajoutée goutte à goutte une solution de 3,5-dioxo-(2H,4H)-1,2,4-triazine (25 g ; 0,22 mole) dans le DMF (350 ml). Après 30 minutes d'agitation à température ambiante, on ajoute l'iodure de méthyle (27,4 ml) et maintien une nuit sous agitation. Après concentration à sec sous vide, le résidu est repris dans le DMF (300 ml) et additionné d'hydrure de sodium à 60 % (8,8 g ; 0,22 mole). Après 4 heures d'agitation, on ajoute l'iodure de methyle (27,4 ml) et agite une nuit à température ambiante. Le mélange réactionnel est concentré à sec sous vide, repris par une solution aqueuse saturée en NaCl (100 ml) et extrait par l'acétate d'éthyle (5 x 200 ml). Les phases organiques groupées sont séchées (Na₂SO₄) et concentrées à sec sous vide. On obtient après cristallisation, lavage à l'eau et séchage sous vide le composé 1a (16,43 g).
   F = 64°C
   CCM : gel de silice 60 F 254 Merck
   CH₂ Cl₂ - Acétate d'éthyle 70.30 RF : 0.53
b) 6-bromo-2,4 diméthyl-3,5-dioxo-(2H,4H)-1,2,4-triazine 1b.
   Le composé 1a (13,3 g) est traité dans l'eau (100 ml) par le brome (18 ml) pendant 12 heures à 60°C. Après évaporation sous vide, le mélange réactionnel est extrait à l'acétate d'éthyle (2 x 100 ml). Les phases organiques, groupées, sont séchées (Mg SO₄) et évaporées à sec sous vide.
   On obtient après recristallisation dans l'éther éthylique le composé 1b (7,8 g) utilisé tel quel dans l'étape suivante.
   c) 2,4-diméthyl-3,5-dioxo[2H,4H]-6-[4-(4-(3-trifluorométhylphényl)pipérazino) butylamino]-1,2,4-triazine 1.
   Le composé 1b (1 g) et la 4-(4-(3-trifluorométhylphenyl)pipérazino)butylamine (1,4 g) sont chauffés pendant 3 heures au reflux du n-butanol (30 ml) en présence de triéthylamine (2 ml). Ce mélange réactionnel est concentré sous vide, repris par la soude 1N et extrait au dichlorométhane (2 x 100 ml). Ces phases organiques séchées (Na₂SO₄) sont concentrées sous vide et chromatographiées sur gel de silice (87 g). Le mélange éther isopropylique/dioxane/triéthylamine 80/15/5 élue le composé 1 qui est purifié, après évaporation du solvant, par recristallisation dans l'éther isopropylique.
   F = 87°C
   CCM : gel de silice 60 F 254 Merck
   Ether isopropylique - dioxane - triéthylamine 80-15-5
   Rf = 0,3.

### Exemple 2 :

### 2,4-diméthyl-3,5-dioxo-(2H,4H)-6-(4-(4-(3-chlorophényl) pipérazino) butylamino)-1,2,4-triazine 2.

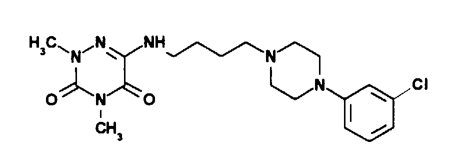

Ce composé est préparé selon le procédé décrit dans l'exemple 1, en utilisant au stade c) la 4-(4-(3-chlorophényl)pipérazino)butylamine.
F = 122°C
CCM : gel de silice 60 F 254 Merck
Ether isopropylique - dioxane - triéthylamine 80-15-5
Rf = 0,3.

### Exemple 3 :

### 2,4-diméthyl-3,5-dioxo-(2H,4H)-6-(4-(4-(2-méthoxyphényl)pipérazino)butylamino)-1,2,4-triazine 3.

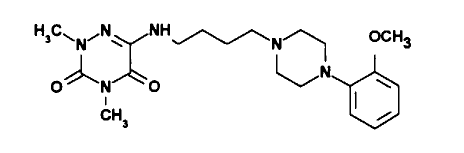

Ce composé est préparé selon le procédé décrit à l'exemple 1, en utilisant au stade c) la 4-(4-(2-méthoxyphényl)pipérazino)butylamine.
F = 122°C
CCM : gel de silice 60 F 254 Merck
Ether isopropylique - dioxane - triéthylamine 80-15-5
Rf = 0,3.

### Exemple 4 :

### 2,4-diméthyl-3,5-dioxo-(2H,4H)-6-(4-(4-(4-chlorophényl)pipérazino) butylamino)-1,2,4-triazine 4.

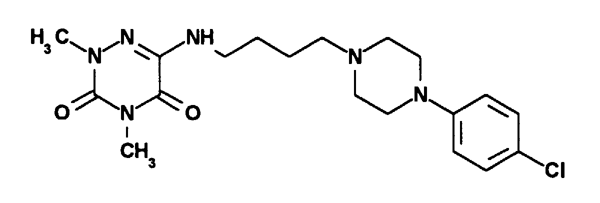

Ce composé est préparé selon le procédé décrit à l'exemple 1, en utilisant au stade c) la 4-(4-(4-chlorophényl)pipérazino)butylamine.
F = 102°C
CCM : gel de silice 60 F 254 Merck
Ether isopropylique - dioxane - triéthylamine 80-15-5
Rf = 0,35.

### Exemple 5 :

### 2,4-diméthyl-3,5-dioxo-(2H,4H)-6-(4-(4-(2-pyrimidyl)pipérazino)butylamino)-1,2,4-triazine.

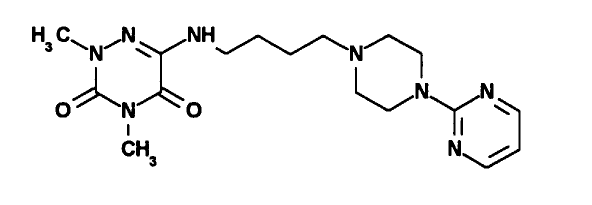

Ce composé est préparé selon le procédé décrit à l'exemple 1 en utilisant au stade c) la 4-(4-(2-pyrimidyl)pipérazino)butylamine.
F = 120°C
CCM : gel de silice 60 F 254 Merck
Ether isopropylique - dioxane - triéthylamine 85-10-5
Rf = 0,2.

### Exemple 6 :

### 2,4-diméthyl-3,5 dioxo-(2H,4H)-6-(3-(4-(3-trifluorométhylphényl)pipérazino) pro- pylamino)-1,2,4-triazine 6.

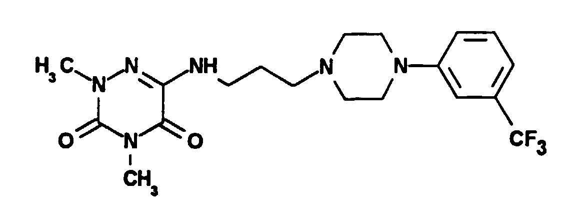

Ce composé est préparé selon le procédé décrit à l'exemple 1 en utilisant au stade c) la 3-(4-(3-trifluorométhylphényl)pipérazino)propylamine.
F = 109°C
CCM : gel de silice 60 F 254 Merck
Ether isopropylique - dioxane - triéthylamine 85-10-5
Rf = 0,3.

### Exemple 7 :

### 2,4-diméthyl-3,5 dioxo-(2H,4H)-6-(2-(4-(3-trifluorométhylphényl)pipérazino) éthylamino)-1,2,4-triazine 7.

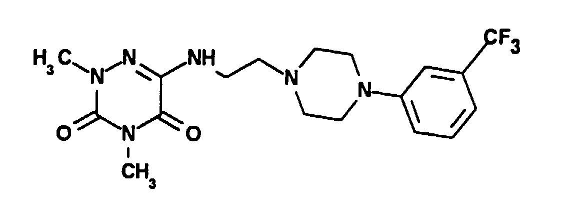

Ce composé est préparé selon le procédé décrit à l'exemple 1 en utilisant au stade c) la 2-(4-(3-trifluorométhylphényl)pipérazino)éthylamine.
F = 90°C
CCM : gel de silice 60 F 254 Merck
Ether isopropylique - dioxane - triethylamine 85-10-5
Rf = 0,33.

### Exemple 8 :

### 2,4-dibutyl-3,5-dioxo-(2H, 4H)-6-(3-(4-(3-trifluorométhylphényl)pipérazino) propylamino)-1,2,4-triazine 8.

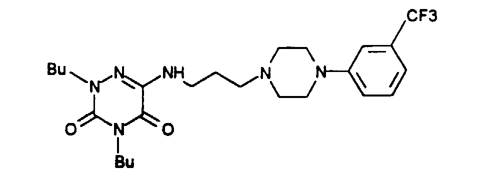

Ce composé est préparé selon le procédé décrit à l'exemple 1 en utilisant au stade a le bromure de butyle et au stade c la 3-(4-(3-trifluorométhylphényl)pipérazino) propylamine.
F = 66°C
CCM : gel de silice 60 F 254 Merck
Ether isopropylique - dioxane - triéthylamine 85-15-5
Rf = 0,48.

### Exemple 9 :

### 2,4-diméthyl-3,5-dioxo-(2H, 4H)-6-(4-(1,4-benzodioxan-2-ylméthylamino) butylamino)-1,2,4-triazine 9.

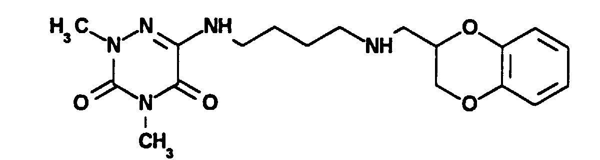

Ce composé est préparé selon le procédé décrit à l'exemple 1 en utilisant au stade c) la 4-(1,4-benzodioxan-2-ylméthylamino)butylamine.
F = 214°C (chlorhydrate)
CCM : gel de silice 60 F 254 Merck
Ether éthylique - dioxane - triéthylamine 80-15-5
Rf = 0,2.

### Exemple 10 :

### 2,4-diméthyl-3,5-dioxo-(2H, 4H)-6-(3-(1,4-benzodioxan-2-ylméthylamino) propylamino)-1,2,4-triazine 10.

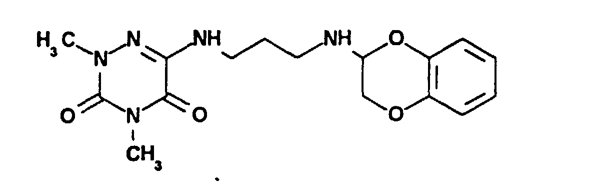

Ce composé est préparé selon le procédé décrit à l'exemple 1 en utilisant au stade c) la 3-(1,4-benzodioxan-2-ylméthylamino)propylamine.
F = 120°C
CCM : gel de silice 60 F 254 Merck
Chloroforme - Méthanol 90-10
Rf = 0,42.

### Exemple 11 :

### 4-butyl-3,5-dioxo-(2H,4H)-6-(4-(4-(3-trifluorométhylphényl)pipérazino)butylamino)-1,2,4-triazine 11.

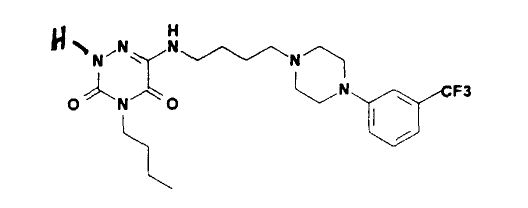

**a) 2-acétyl-3,5-dioxo-(2H,4H)-1,2,4-triazine 11a.**
   La 3,5-dioxo-(2H,4H)-1,2,4-triazine (48,88 g) est chauffée au reflux de l'anhydride acétique (300 ml) pendant 90 minutes. Après refroidissement et concentration à sec sous vide, le résidu est repris au toluène (300 ml) et refroidit à 0°C pendant 2 heures. Le précipité blanc formé est filtré, essoré et séché sous vide à 70°C pour donner le composé 11a (57,6 g).
   F = 150°C
   CCM : gel de silice 60 F 254 Merck
   CH₂Cl₂-MeOH : 85-15
   Rf = 0,61.
**b) 4-butyl-3,5-dioxo-(2H,4H)-1,2,4-triazine 11b.**
   A une suspension d'hydrure de sodium à 50 % (4,8 g) dans de l'huile dans le DMF (100 ml), on ajoute goutte à goutte, en maintenant la température à l'ambiante le composé 11a (15,5 g) en solution dans le DMF (200 ml). Après 2 heures d'agitation on ajoute le bromobutane (20 g) et maintien l'agitation pendant 12 heures. Le mélange réactionnel est concentré à sec sous vide à 60°C, repris à l'eau puis extrait au chlorure de méthylène. Après concentration à sec sous vide, l'huile est reprise à l'éthanol (100 ml) puis additionnée d'acide p. toluène sulfonique (2 g). Après chauffage à reflux 2 heures et abandon à température ambiante pendant 12 heures, le mélange réactionnel est filtré. Le précipité est lavé avec une solution aqueuse de bicarbonate de sodium et à l'eau puis séché à l'étuve sous vide pour donner le composé 11b (8,7 g).
   F = 135°C
   CCM : gel de silice 60 F 254 Merck
   CHCl₃-MeOH : 9-1
   Rf = 0,35.
**c) 4-butyl-3,5-dioxo-(2H,4H)-6-(4-(4-(3-trifluorométhylphényl)pipérazino) butylamino)-1,2,4-triazine 11**
   Le composé 11b est traité de manière identique à celle décrite dans l'exemple 1b et 1c pour conduire au composé 11.
   F = 140°C
   CCM : gel de silice 60F254 Merck
   Ether isopropylique - dioxane - triéthylamine 80-15-5
   Rf = 0,45.

### Exemple 12 :

### 4-butyl-3,5-dioxo-(2H,4H)-6-(3-(4-(3-trifluorométhylphényl)pipérazino)propylamino)-1,2,4-triazine 12.

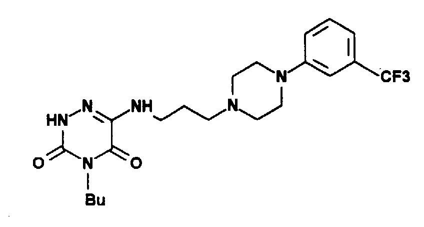

Ce composé est préparé selon le procédé décrit à l'exemple 11 en utilisant au dernier stade la 3-(4-(3-trifluorométhylphényl)pipérazino)propylamine.
F = 154°C
CCM : gel de silice 60 F 254 Merck
Ether isopropylique - dioxane - triéthylamine 80-15-5
Rf = 0,40.

### Exemple 13 :

### 4-butyl-3,5-dioxo-(2H,4H)-6-(2-(4-(3-trifluorométhylphényl)pipérazino)éthylamino)-1,2,4-triazine 13.

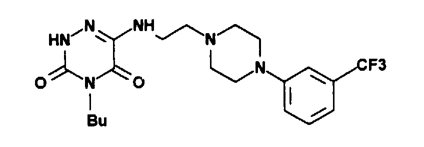

Ce composé est préparé selon le procédé décrit à l'exemple 11 en utilisant au dernier stade la 2-(4-(3-trifluorométhylphényl)pipérazino)éthylamine.
F = 149°C
CCM : gel de silice 60 F 254 Merck
Ether isopropylique - dioxane - triéthylamine 80-15-5
Rf = 0,35.

### Exemple 14 :

### 2-méthyl-3,5-dioxo-(2H,4H)-6-(4-(4-(3-trifluorométhylphényl)pipérazino) butyl- amino)-1,2,4-triazine 14.

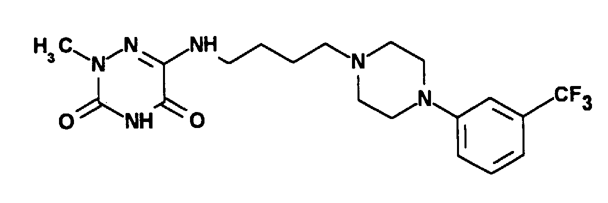

a) 3-thioxo-5-oxo-(2H,4H)-1,2,4-triazine 14a.
   A une solution d'acide glyoxylique à 50 % (148 g ; 110 ml) dans l'eau (1 litre), on ajoute une solution de thiosemicarbazide (91,1 g) dans l'eau (1 litre) à 70°C. Après 1 heure sous agitation, le mélange est abandonné 12 heures à 0°C. Le précipité formé est filtré, essoré et séché sous vide en présence de P₂O₅ à 70°C.
   Le composé ainsi obtenu (112 g) est repris par la soude N (1500 ml) et porté 3 heures à reflux. Après refroidissement à température ambiante, on ajoute goutte à goutte l'acide acétique (91 ml) et abandonne 1 nuit. Le précipité formé est filtré, essoré et séché sous vide à 80°C en présence de P₂O₅ pour donner le composé 14a (62 g).
   F = 260°C
b) 3-méthylthio-5-oxo-(2H)-1,2,4 triazine 14b.
   Le composé 14a (62 g) en solution dans la soude 2 N (480 ml) est traité par une solution d'iodure de méthyle (30 ml). Après 2 heures à température ambiante sous agitation, on ajoute goutte à goutte l'acide acétique (36 ml) et abandonne 1 nuit à 0°C. Le précipité formé est filtré, essoré et séché sous vide à 60°C en présence de P₂O₅ pour donner le composé 14b (56,4 g).
   F = 215°C
c) 2-méthyl-3,5-dioxo-(2H,4H)-1,2,4-triazine 14c.
   Le composé 14b (14,3 g) en solution dans le DMF (140 ml) est ajouté à une suspension de NaH à 60 % (4,2 g) dans de l'huile dans le DMF (40 ml).
   Après 2 heures d'agitation à température ordinaire, on ajoute l'iodure de méthyle (7,5 ml) et agite 12 heures supplémentaires. Après concentration sous vide, le résidu est repris à l'eau (20 ml) et extrait au chlorure de méthylène (2 x 50 ml). Les phases organiques, séchées (Na₂SO₄) sont concentrées à sec sous vide. L'huile brune ainsi obtenue (14,2 g) est traitée par l'acide chlorhydrique 2N (80 ml) pendant 30 minutes à 100°C. Après refroidissement, le mélange réactionnel est extrait au chlorure de méthylène (2 x 50 ml). Les phases organiques sont séchées (Na₂SO₄) et concentrées sous vide. Le résidu est repris à l'éther éthylique chaud (100 ml), traité au charbon actif et concentré à sec sous vide. Par recristallisation dans le toluène, on obtient, après séchage à 60°C sous vide, le composé 14c (2,6 g).
   F = 118°C.
d) 2-méthyl-3,5-dioxo-(2H,4H)-6-(4-(4-(3-trifluorométhylphényl)pipérazino)butyl -amino)-1,2,4-triazine 14.
   Le composé 14c est traité de manière identique à celle décrite dans l'exemple 1b et lc pour conduire au composé 14.
   CCM : gel de silice 60 F 254 Merck
   Ether isopropylique - dioxane - triéthylamine 80-15-5
   Rf = 0,40

### Exemple 15 :

### 3,5-dioxo-(2H,4H)-6-(4-(4-(3-trifluorométhylphényl)pipérazino)butylamino)-1,2,4-triazine 15.

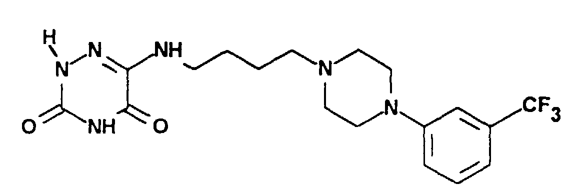

a) 2-acétyl-6-bromo-3,5-dioxo-(2H,4H)-1,2,4-triazine 15a.
   La 6-bromo-3,5-dioxo-(2H,4H)-1,2,4-triazine (5 g), obtenue à partir de la 3,5-dioxo-(2H,4H)-1,2,4-triazine selon la technique décrite à l'exemple 1b, est traitée par l'anhydride acétique (50 ml) pendant 7 heures à reflux. Après concentration à sec sous vide et reprise à l'éther éthylique, le précipité est filtré, essoré et séché sous vide à 50°C pour donner le composé 15a (4,6 g).
b) 3,5-dioxo-(2H,4H)-6-(4-(4-(3-trifluorométhylphényl)pipérazino)butylamino)-1,2,4-triazine 15.
   Le composé 15a (2,44 g) en solution dans le butanol (15 ml) est traité par la 4-(4-(3-trifluorométhylphényl)pipérazino)butylamine (2,88 g) en présence de triéthylamine (1,5 ml) à reflux pendant 8 heures. Après concentration à sec sous vide et reprise à l'éthanol (50 ml), on ajoute l'acide p-toluène sulfonique (1,5 g) et traité 4 heures à reflux. Le mélange réactionnel est concentré à sec sous vide, repris par l'eau (100 ml) et extrait à l'acétate d'éthyle (3 x 200 ml). Les phases organiques séchées (Na₂SO₄) sont concentrées et chromatographiées sur silice. Le mélange CH₂Cl₂-MeOH 90-10 fournit, après concentration à sec sous vide, le dérivé 15 (1,26 g).
   F = 94°C
   CCM : gel de silice 60 F 254 Merck
   CH₂Cl₂ - MeOH - NH₄OH 90-9-1
   Rf = 0.46
   Les composés de l'invention ont été soumis à des essais pharmacologiques qui ont mis en évidence leur intérêt comme substances actives en thérapeutique. Ainsi, ils ont fait l'objet d'une étude portant sur leur affinité pour les récepteurs sérotoninergiques du type 5-HT_{1A}.

L'étude de la liaison au récepteur 5-HT_{1A} est réalisée comme décrit par Sleight et Peroutka (Naunyn-Schmiedebergs Arch. Pharmacol., 343, 106-116, 1991). Pour ces expérimentations, des cortex cérébraux de rat sont utilisés. Le cerveau est disséqué et le cortex est homogénéisé dans 20 volumes de tampon Tris-HCl (50 mM, pH 7,4 à 25°C) maintenu à 4°C. L'homogénat est centrifugé à 39000 x g pendant 10 minutes, le culot de centrifugation est mis en suspension dans le même volume de tampon et centrifugé à nouveau. Après une nouvelle mise en suspension dans les mêmes conditions, l'homogénat est incubé pendant 10 minutes à 37°C puis centrifugé à nouveau. Le culot final est mis en suspension dans 80 volumes de tampon de réaction contenant : pargyline (10⁻⁵M), CaCl₂ (4 mM) et acide ascorbique (0,1 %) dans du Tris-HCl (50 mM, pH 7,4 à 25°C). La concentration finale de tissu dans le milieu d'incubation est 10 mg/tube.
Dans les expériences de saturation, les tubes de réaction contiennent 0,1 ml de différentes concentrations de [³H]8-OH-DPAT (comprises entre 0,06 et 8 nM), 0,1 ml de tampon de réaction ou de 5-HT (10⁻⁵M, pour déterminer la liaison non-spécifique) et 0,8 ml de tissu.

Les expériences de déplacement sont réalisées comme décrit par Sleight et Peroutka (Naunyn-Schmiedebergs Arch. Pharmacol., 343, 106-116, 1991). Toutes les dilutions de produits à étudier sont réalisées dans le tampon de réaction. Les tubes de réaction contiennent 0,1 ml de [³H]8-OH-DPAT (0,2 nM), 0,1 ml de produit à tester 6-7 concentrations (dilutions successives au 1/10) et 0,8 ml de tissu. Si l'affinité présumée des produits se situe dans le domaine nanomolaire, la plus faible concentration testée est 10⁻¹¹M, si le produit a une affinité présumée faible, la plus forte concentration testée est 10⁻⁴M. Les tubes de réaction sont incubés à 23°C pendant 30 minutes puis rapidement filtrés sous vide sur filtres Whatman GF/B, les tubes sont rinçés avec 2 x 5 ml de tampon Tris-HCl (50 mM, pH 7, 4 à 25°C). La radioactivité recueillie sur le filtre est analysée en scintillation liquide en ajoutant 4 ml de liquide scintillant (Emulsifer Safe, Packard). Toutes les expériences sont réalisées en triple et répétées au moins 3 fois.

La constante de dissociation (K_{D}) et le nombre maximum de sites de liaison (Bmax) pour le radioligand sont estimés à partir des expériences de saturation en utilisant le programme de régression non-linéaire EBDA/LIGAND (Biosoft) (Munson et Rodbard, Anal, Biochem., 107, 220-239, 1980). Les constantes d'affinité (Ki) des produits de référence sont estimées à partir des expériences de déplacement en utilisant le programme de régression non-linéaire EBDA/LIGAND. Cette méthode admet que la valeur du coefficient de Hill n'est pas différente de l'unité. Les données des expériences de déplacement sont analysées respectivement avec les modèles un site et deux sites et le F calculé permet de déterminer si le modèle deux sites est plus représentatif des données obtenues que le modèle un site. Les valeurs de pKi sont données sous forme de moyenne ± SEM de 3 à 5 expériences.

Le tableau 2 donne, à titre d'exemple, les pKi 5-HT_{1A} pour certains dérivés de l'invention, par rapport à la Buspirone qui est utilisée en clinique.

**Tableau 2 :**

| affinité pour le récepteur 5-HT_{1A} | |
|---|---|
| Composé n° | pKi |
| 1 | 9.50 |
| 2 | 9.40 |
| 3 | 9.21 |
| 6 | 7.79 |
| 9 | 8.57 |
| 10 | 8.42 |
| 11 | 7.90 |
| Buspirone | 7.95 |

Les résultats des essais montrent que les composés de formule générale 1 possèdent une haute affinité pour les récepteurs sérotoninergiques de type 5-HT_{1A}.

L'activité centrale des composés de l'invention a été évaluée par leur capacité de provoquer le syndrome 5-HT, qui est caractérisé par une flexion et une extension alternées des pattes avant (reciprocal fore-paw treading : FPT), la rétraction de la lèvre inférieure (lower-lip retraction : LLR) et par une posture où la surface ventrale de l'animal est en contact avec le sol de la cage avec les pattes arrières étendues (flat body posture : FBP).

Les expériences de l'évaluation du syndrome 5-HT sont réalisées chez le rat mâle (Sprague Dawley) selon la méthode décrite par F.C. Colpaert et al. (Drug Dev. Res., 26, 21-48; 1992).

Le tableau 3 donne, à titre d'exemple, les doses actives (ED₅₀) pour certains dérivés de l'invention par rapport à un produit de référence, la Buspirone.

**Tableau 3 :**

| Syndrome 5-HT | | | |
|---|---|---|---|
| Composé n° | ED₅₀ : mg/kg ip | | |
| | FBP | LLR | FPT |
| | | | |
| 1 | 0.31 | 0.31 | 0.31 |
| | | | |
| 2 | 0.31 | 0.31 | 40 |
| | | | |
| Buspirone | 5.0 | 1.25 | > 40 |
| | | | |

Les résultats des essais montrent que les composés de formule générale 1 possèdent, in vitro, une haute affinité pour les récepteurs sérotoninergiques de type 5-HT_{1A}. In vivo, ils montrent une activité agoniste au niveau de ces récepteurs.

Les composés de l'invention peuvent donc être utiles pour le traitement de l'anxiété, la dépression, les troubles de sommeil, pour la régularisation de la prise de nourriture, pour la régulation de la sécrétion gastrique, et pour le traitement des désordres vasculaires, cardiovasculaires et cérébrovasculaires tels que l'hypertention ou la migraine.
Les préparations pharmaceutiques contenant ces principes actifs peuvent être mises en forme pour l'administration par voie orale, rectale ou parentérale, par exemple sous la forme de capsules, comprimés, granulés, gélules, solutés liquides, sirops ou suspensions buvables, et contenir les excipients appropriés.

Il est également possible d'y associer d'autres principes actifs pharmaceutiquement et thérapeutiquement acceptables.

## Revendications

1. Dérivés de 3,5-dioxo-6-amino-(2H,4H)-1,2,4-triazine correspondant à la formule générale I. 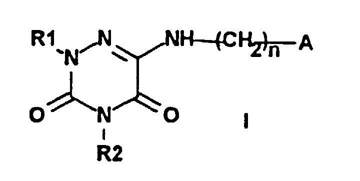 dans laquelle :
- R₁ et R₂, identiques ou différents représentent l'hydrogène ou un radical alcoyle en C₁-C₆
- n peut prendre les valeurs entières de 2 à 6
- A représente un groupement de type
. aryl piperazino II 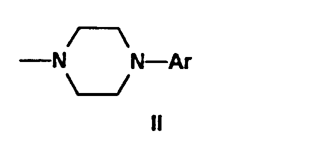 le groupement Ar représentant lui-même une structure aromatique: phényle, napthyle, pyrimidyle, ou pyridyle, éventuellement substituée par un ou plusieurs groupements: alcoyle en C₁-C₃, alcoxy en C₁-C₃, hydroxy, trifluorométhyle ou halogène.
. benzodioxanyl méthyl amino ou pyridodioxanyl methyl amino III 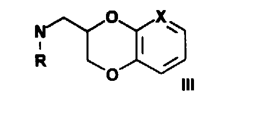 dans lequel R représente l'hydrogène ou un groupement alcoyle en C₁-C₃ et X représente un atome d'azote ou de carbone ainsi que les sels organiques ou minéraux thérapeutiquement acceptables de ces molécules et les énantiomères pour celles présentant un carbone asymétrique.

2. Composés de formule générale I selon la revendication 1 caractérisés en ce que ils sont choisis parmi :
- 2,4-diméthyl-3,5-dioxo-(2H,4H)-6-(4-(4-(3-trifluorométhylphényl) pipérazino)butylamino)-1,2,4-triazine.
- 2,4-diméthyl-3,5-dioxo-(2H,4H)-6-(4-(4-(3-chlorophényl)pipérazino)butylamino)-1,2,4-triazine.
- 2,4-diméthyl-3,5-dioxo-(2H,4H)-6-(4-(4-(2-méthoxyphényl)pipérazino)butylamino)-1,2,4-triazine.
- 2,4-diméthyl-3,5-dioxo-(2H,4H)-6-(4-(4-(4-chlorophényl)pipérazino)butylamino)-1,2,4-triazine.
- 2,4-diméthyl-3,5-dioxo-(2H,4H)-6-(4-(4-(2-pyrimidyl)pipérazino) butylamino)-1,2,4-triazine.
- 2,4-diméthyl-3,5-dioxo-(2H,4H)-6-(3-(4-(3-trifluorométhylphényl) pipérazino) propylamino)-1,2,4-triazine.
- 2,4-diméthyl-3,5-dioxo-(2H,4H)-6-(2-(4-(3-trifluorométhylphényl) pipérazino) éthylamino)-1,2,4-triazine.
- 2,4-dibutyl-3,5-dioxo-(2H,4H)-6-(3-(4-(3-trifluorométhylphényl)pipérazino)propylamino)-1,2,4-triazine.
- 2,4-diméthyl-3,5-dioxo-(2H,4H)-6-(4-(1,4-benzodioxan-2-ylméthylamino)butylamino)-1,2,4-triazine.
- 2,4-diméthyl-3,5-dioxo-(2H,4H)-6-(3-(1,4-benzodioxan-2-ylméthylamino) propylamino)-1,2,4-triazine.
- 4-butyl-3,5-dioxo-(2H,4H)-6-(4-(4-(3-trifluorométhylphényl)pipérazino) butylamino)-1,2,4-triazine.
- 4-butyl-3,5-dioxo-(2H,4H)-6-(3-(4-(3-trifluorométhylphényl)pipérazino) propylamino)-1,2,4-triazine.
- 4-butyl-3,5-dioxo-(2H,4H)-6-(2-(4-(3-trifluorométhylphenyl)pipérazino) éthylamino)-1,2,4-triazine.
- 2-méthyl-3,5-dioxo-(2H,4H)-6-(4-(4-(3-trifluorométhylphényl)pipérazino) butylamino)-1,2,4-triazine.
- 3,5-dioxo-(2H,4H)-6-(4-(4-(3-trifluorométhylphényl)pipérazino)butylamino) -1,2,4-triazine.

3. Procédé de préparation des composés chimiques selon les revendications 1 et 2, caractérisé en ce que l'on obtient les composés de formule I par réaction d'une 6-bromo-3,5-dioxo-(2H,4H)-1,2,4-triazine IV avec une amine de formule V. 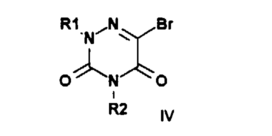
H₂N-(CH2)n-A V
R₁, R₂, n, A ayant la même signification que dans les revendications 1 et 2.

4. Procédé de préparation des composés chimiques selon la revendication 3 caractérisé en ce que la réaction entre les composés IV et V est effectuée au reflux du butanol en présence d'une base telle que la triethylamine.

5. A titre de médicaments utilisables dans le traitement des maladies nécessitant des agonistes de récepteurs SHT_{1A}, les composés définis selon l'une des revendications 1 et 2.

6. A titre de médicaments utiles, par exemple, pour le traitement de l'anxiété, la dépression, les troubles du sommeil, la régularisation de prise de nourriture, la régulation de la sécrétion gastrique, le traitement des désordres vasculaires, cardiovasculaires et cérébrovasculaires, les composés définis selon l'une des revendications 1 et 2.

7. Composition pharmaceutique caractérisée en ce qu'elle contient un composé défini selon l'une des revendications 1 et 2.

8. Composition pharmaceutique caractérisée en ce qu'elle contient un composé défini selon l'une des revendications 1 et 2 en association avec tout excipient approprié.

9. Composition pharmaceutique caractérisée en ce qu'elle contient un composé défini selon l'une des revendications 1 et 2 associé à un autre principe actif.

## Patentansprüche

1. Derivate von 3,5-Dioxo-6-amino-(2H,4H)-1,2,4-triazin entsprechend der allgemeinen Formel I 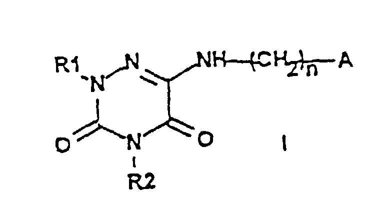 worin:
- R₁ und R₂ identisch oder unterschiedlich sind und Wasserstoff oder einen C₁-C₆ Alkylrest darstellen
- n eine ganze Zahl von 2 bis 6 annehmen kann
- A eine Gruppe vom Typ
Arylpiperazino II 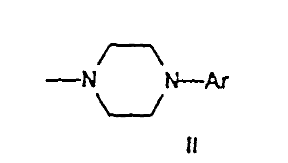 darstellt, worin die Gruppe Ar selbst eine aromatische Phenyl-, Naphthyl-, Pyrimidyl- oder Pyridylstruktur darstellt, welche gegebenenfalls durch eine oder mehrere C₁-C₃ Alkyl-, C₁-C₃ Alkoxy-, Hydroxy-, Trifluormethyl- oder Halogengruppen substituiert ist,
Benzodioxanylmethylamino oder Pyridodioxanylmethylamino III darstellt, 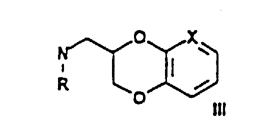 worin R Wasserstoff oder eine C₁-C₃ Alkylgruppe darstellt und X ein Stickstoffatom oder ein Kohlenstoffatom darstellt, sowie die therapeutisch akzeptablen organischen oder anorganischen Salze dieser Moleküle und die Enantiomeren von denen, die ein asymmetrisches Kohlenstoffatom aufweisen.

2. Verbindungen der allgemeinen Formel I nach Anspruch 1, dadurch gekennzeichnet,
daß sie ausgewählt sind aus:
- 2,4-Dimethyl-3,5-dioxo-(2H,4H)-6-(4-(4-(3-trifluormethylphenyl)piperazino)butylamino)-1,2,4-triazin.
- 2,4-Dimethyl-3,5-dioxo-(2H,4H)-6-(4-(4-(3-chlorphenyl)piperazino)butylamino)-1,2,4-triazin.
- 2,4-Dimethyl-3,5-dioxo-(2H,4H)-6-(4-(4-(2-methoxyphenyl)piperazino)butylamino)-1,2,4-triazin.
- 2,4-Dimethyl-3,5-dioxo-(2H,4H)-6-(4-(4-(4-chlorphenyl)piperazino)butylamino)-1,2,4-triazin.
- 2,4-Dimethyl-3,5-dioxo-(2H,4H)-6-(4-(4-(2-pyrimidyl)piperazino)butylamino)-1,2,4-triazin.
- 2,4-Dimethyl-3,5-dioxo-(2H,4H)-6-(3-(4-(3-trifluormethylphenyl)piperazino)propylamino)-1,2,4-triazin.
- 2,4-Dimethyl-3,5-dioxo-(2H,4H)-6-(2-(4-(3-trifluormethylphenyl)piperazino)ethylamino)-1,2,4-triazin.
- 2,4-Dibutyl-3,5-dioxo-(2H,4H)-6-(3-(4-(3-trifluormethylphenyl)piperazino)propylamino)-1,2,4-triazin.
- 2,4-Dimethyl-3,5-dioxo-(2H,4H)-6-(4-(1,4-benzodioxan-2-ylmethylamino)butylamino)-1,2,4-triazin.
- 2,4-Dimethyl-3,5-dioxo-(2H,4H)-6-(3-(1,4-benzodioxan-2-ylmethylamino)propylamino)-1,2,4-triazin.
- 4-Butyl-3,5-dioxo-(2H,4H)-6-(4-(4-(3-trifluormethylphenyl)piperazino)butylamino)-1,2,4-triazin.
- 4-Butyl-3,5-dioxo-(2H,4H)-6-(3-(4-(3-trifluormethylphenyl)piperazino)propylamino)-1,2,4-triazin.
- 4-Butyl-3,5-dioxo-(2H,4H)-6-(2-(4-(3-trifluormethylphenyl)piperazino)ethylamino)-1,2,4-triazin.
- 2-Methyl-3,5-dioxo-(2H,4H)-6-(4-(4-(3-trifluormethylphenyl)piperazino)butylamino)-1,2,4-triazin.
- 3,5-dioxo-(2H,4H)-6-(4-(4-(3-trifluormethylphenyl) piperazino)butylamino)-1,2,4-triazin.

3. Verfahren zur Herstellung der chemischen Verbindungen nach Anspruch 1 und 2,
dadurch gekennzeichnet,
daß man die Verbindung der Formel I durch Umsetzen eines 6-Brom-3,5-dioxo-(2H,4H)-1,2,4-triazins IV mit einem Amin der Formel V erhält, 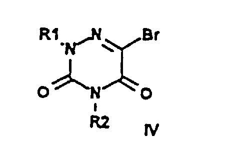
H₂N-(CH2)n-A V
worin R₁, R₂, n, A die gleiche Bedeutung wie in den Ansprüchen 1 und 2 haben.

4. Verfahren zur Herstellung der chemischen Verbindungen nach Anspruch 3,
dadurch gekennzeichnet,
daß die Umsetzung zwischen den Verbindungen IV und V unter Rückfluß in Butanol in Gegenwart einer Base, wie etwa Triethylamin, bewirkt wird.

5. Verbindungen nach einem der Ansprüche 1 und 2 als Medikamente, welche zur Behandlung von Erkrankungen geeignet sind, die Antagonisten des Rezeptors 5HT_{1A} erfordern.

6. Verbindungen nach einem der Ansprüche 1 und 2 als Medikamente, welche z.B. für die Behandlung von
Angstzuständen, Depressionen, Schlafschwierigkeiten, für die Regulierung der Nahrungsaufnahme, die Regulierung der Magenabsonderung, die Behandlung von Gefäß-, Herzgefäß- und Gehirngefäßstörungen verwendet werden.

7. Pharmazeutische Zusammensetzung,
dadurch gekennzeichnet,
daß sie eine Verbindung nach einem der Ansprüche 1 oder 2 enthält.

8. Pharmazeutische Zusammensetzung,
dadurch gekennzeichnet,
daß sie eine Verbindung nach einem der Ansprüche 1 oder 2 zusammen mit jeder beliebigen geigneten Grundmasse enthält.

9. Pharmazeutische Zusammensetzung,
dadurch gekennzeichnet,
daß sie eine Verbindung nach einem der Ansprüche 1 oder 2 zusammen mit einem anderen Wirkstoff enthält.

## Claims

1. 3,5-Dioxo-6-amino-(2H,4H)-1,2,4-triazine compounds corresponding to the general formula I 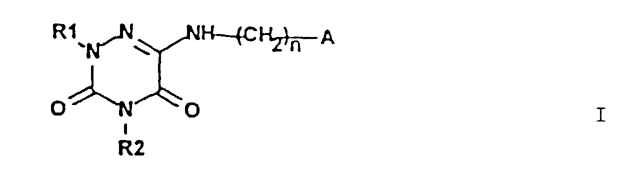 wherein:
- R₁ and R₂, which are identical or different, represent hydrogen or a C₁-C₆alkyl radical
- n may be an integer of from 2 to 6
- A represents a group of the type
. arylpiperazino II 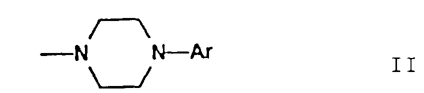 the group Ar itself representing an aromatic phenyl, naphthyl, pyrimidyl or pyridyl structure that is optionally substituted by one or more groups: C₁-C₃alkyl, C₁-C₃alkoxy, hydroxy, trifluoromethyl or halogen;
. benzodioxanylmethylamino or pyridodioxanylmethylamino III 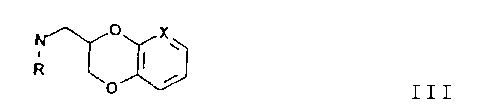 wherein R represents hydrogen or a C₁-C₃alkyl group and X represents a nitrogen or carbon atom,
and also the therapeutically acceptable organic or mineral salts of those molecules and the enantiomers thereof having an asymmetric carbon atom.

2. Compounds of the general formula I according to claim 1, characterised in that they are selected from :
- 2,4-dimethyl-3,5-dioxo-(2H,4H)-6-(4-(4-(3-trifluoromethylphenyl)piperazino)butylamino)-1,2,4-triazine,
- 2,4-dimethyl-3,5-dioxo-(2H,4H)-6-(4-(4-(3-chlorophenyl)piperazino)butylamino)-1,2,4-triazine,
- 2,4-dimethyl-3,5-dioxo-(2H,4H)-6-(4-(4-(2-methoxyphenyl)piperazino)butylamino)-1,2,4-triazine,
- 2,4-dimethyl-3,5-dioxo-(2H,4H)-6-(4-(4-(4-chlorophenyl)piperazino)butylamino)-1,2,4-triazine,
- 2,4-dimethyl-3,5-dioxo-(2H,4H)-6-(4-(4-(2-pyrimidyl)piperazino)butylamino)-1,2,4-triazine,
- 2,4-dimethyl-3,5-dioxo-(2H,4H)-6-(3-(4-(3-trifluoromethylphenyl)piperazino)propylamino)-1,2,4-triazine,
- 2,4-dimethyl-3,5-dioxo-(2H,4H)-6-(2-(4-(3-trifluoromethylphenyl)piperazino)ethylamino)-1,2,4-triazine,
- 2,4-dibutyl-3,5-dioxo-(2H,4H)-6-(3-(4-(3-trifluoromethylphenyl)piperazino)propylamino)-1,2,4-triazine,
- 2,4-dimethyl-3,5-dioxo-(2H,4H)-6-(4-(1,4-benzodioxan-2-ylmethylamino)butylamino)-1,2,4-triazine,
- 2,4-dimethyl-3,5-dioxo-(2H,4H)-6-(3-(1,4-benzodioxan-2-ylmethylamino)propylamino)-1,2,4-triazine,
- 4-butyl-3,5-dioxo-(2H,4H)-6-(4-(4-(3-trifluoromethylphenyl)piperazino)butylamino)-1,2,4-triazine,
- 4-butyl-3,5-dioxo-(2H,4H)-6-(3-(4-(3-trifluoromethylphenyl)piperazino)propylamino)-1,2,4-triazine,
- 4-butyl-3,5-dioxo-(2H,4H)-6-(2-(4-(3-trifluoromethylphenyl)piperazino)ethylamino)-1,2,4-triazine,
- 2-methyl-3,5-dioxo-(2H,4H)-6-(4-(4-(3-trifluoromethylphenyl)piperazino)butylamino)-1,2,4-triazine,
- 3,5-dioxo-(2H,4H)-6-(4-(4-(3-trifluoromethylphenyl)piperazino)butylamino)-1,2,4-triazine.

3. Process for the preparation of chemical compounds according to claims 1 and 2, characterised in that the compounds of formula I are obtained by reaction of a 6-bromo-3,5-dioxo-(2H,4H)-1,2,4-triazine IV with an amine of formula V 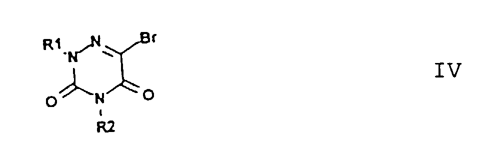
H₂N-(CH₂)ₙ-A V
R₁, R₂, n and A having the same meaning as in claims 1 and 2.

4. Process for the preparation of chemical compounds according to claim 3, characterised in that the reaction between compounds IV and V is carried out at reflux in butanol in the presence of a base, such as triethylamine.

5. The compounds defined in accordance with either claim 1 or claim 2 for use as medicaments in the treatment of disorders requiring 5HT_{1A} receptor agonists.

6. The compounds defined in accordance with either claim 1 or claim 2 for use as medicaments, for example, in the treatment of anxiety, depression or sleep disorders, in the regulation of food intake, the regulation of gastric secretion, and the treatment of vascular, cardiovascular and cerebrovascular disorders.

7. Pharmaceutical composition, characterised in that it contains a compound defined in accordance with either claim 1 or claim 2.

8. Pharmaceutical composition, characterised in that it contains a compound defined in accordance with either claim 1 or claim 2 in association with any appropriate excipient.

9. Pharmaceutical composition, characterised in that it contains a compound defined in accordance with either claim 1 or claim 2 in association with another active ingredient.
